(19) Europäisches Patentamt
European Patent Office
Office européen des brevets

(11) Veröffentlichungsnummer: **0 200 955**
**A2**

# (12) EUROPÄISCHE PATENTANMELDUNG

(21) Anmeldenummer: **86105101.9**

(22) Anmeldetag: **14.04.86**

(51) Int. Cl.[4]: **C07C 79/35**, C07D 307/12, C07C 76/02

(30) Priorität: **27.04.85 DE 3515339**

(43) Veröffentlichungstag der Anmeldung: **17.12.86 Patentblatt 86/46**

(84) Benannte Vertragsstaaten: **BE CH DE FR GB LI**

(71) Anmelder: **CASSELLA Aktiengesellschaft**
**Hanauer Landstrasse 526**
**D-6000 Frankfurt am Main 61(DE)**

(72) Erfinder: **Reh, Kuno, Dr.**
**Lauterbacher Strasse 14**
**D-6000 Frankfurt am Main 61(DE)**
Erfinder: **Schophoff, Friedrich, Dr.**
**Konstanzer Strasse 64**
**D-6000 Frankfurt am Main 61(DE)**

(74) Vertreter: **Urbach, Hans-Georg, Dr.**
**Hanauer Landstrasse 526**
**D-6000 Frankfurt am Main 61(DE)**

(54) **Verfahren zur Herstellung von 2,4-Dinitrophenylethern.**

(57) 2,4-Dinitrophenylether der Formel I

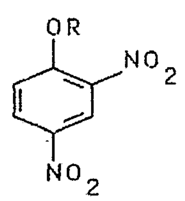

(I)

wobei R z.B. $(C_1$-$C_4)$Alkoxy-$(C_2$-$C_4)$alkyl bedeutet, werden durch Umsetzung eines 2,4-Dinitrochlor- oder -brombenzols mit einem Alkalimetallalkoholat ROM, wobei M ein Alkalimetallkation bedeutet, in einem unpolaren, inerten Lösungsmittel bei einer Temperatur von -25°C bis +50°C hergestellt.

EP 0 200 955 A2

# Verfahren zur Herstellung von 2,4-Dinitrophenylethern

Die Erfindung betrifft ein Verfahren zur Herstellung von 2,4-Dinitrophenylethern der allgemeinen Formel I

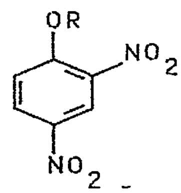

(I)

wobei

R = $(C_1-C_4)$Alkoxy-$(C_2-C_4)$alkyl, Phenoxy-$(C_2-C_4)$-alkyl, $(C_1-C_4)$Alkoxy-$(C_2-C_4)$alkoxy-$(C_2-C_4)$alkyl oder Tetrahydrofurfuryl

bedeutet.

Die Verbindungen der Formel I sind wichtige Vorprodukte, z.B. zur Herstellung von Dispersionsfarbstoffen, wie sie z.B. in der belgische Patentschrift 634 032 beschrieben sind.

Für die Herstellung der Ether der Formel I bieten sich als Ausgangsprodukte z.B. 2,4-Dinitrochlorbenzol und die entsprechenden Alkohole an. Zur Herstellung von 1-(2-Methoxyethoxy)-2,4-dinitrobenzol ist es aus Journal Organic Chemistry USSR 18, (1982), Seite 1087, bekannt, 2,4-Dinitrochlorbenzol mit Natrium-2-methoxy-ethanolat in 2-Methoxyethanol 4 h bei 90°C umzusetzen. Als erhaltene Ausbeute werden dabei jedoch lediglich 62 % angegeben.

Zur Herstellung von 2,4-Dinitro-anisol ist es aus Beispiel 23 der DE-OS 26 34 419 bekannt, 2,4-Dinitrochlorbenzol und Methanol in Gegenwart einer wäßrigen Lösung von Benzyl-dimethyl-laurylammoniumchlorid als Phasentransfer-katalysator umzusetzen. Die Umsetzung von 2,4-Dinitrochlorbenzol mit Methanol in Gegenwart von Natriumhydroxid kann bei Rückflußtemperatur nach der Stufe 1 des Beispiels 1 der EP-PS 0011048 auch ohne Anwesenheit eines Phasentransferkatalysators durchgeführt werden. Bei der Übertragung dieses Verfahrens auf Alkohole der Formel ROH, wobei R die eingangs genannte Bedeutung besitzt, werden jedoch die gewünschten Ether zum Teil nur in mäßigen Ausbeuten erhalten, vor allem treten dabei aber unerwünschte Nebenprodukte, insbesondere 2,4-Dinitrophenol in Mengen von 7 bis 20 % auf.

Hierdurch wird eine aufwendige Abtrennung dieses unerwünschten und störenden Nebenproduktes zwingend erforderlich, weil in Gegenwart von 2,4-Dinitrophenol bei der weiteren Umsetzung - (z.B. Reduktion) der gewünschten Phenolether technisch nicht beherrschbare Probleme der Produktqualität und der Reaktionsführung auftreten, so daß ein angestrebtes Eintopfverfahren auf dieser Basis nicht realisierbar ist. Darüberhinaus gestaltet sich die Abtrennung und Beseitigung des 2,4-Dinitrophenols aufgrund seiner hohen Toxizität ökologisch wie ökonomisch aufwendig.

Das erfindungsgemäße Verfahren zur Herstellung von 2,4-Dinitrophenylethern der allgemeinen Formel I durch Umsetzung eines 2,4-Dinitrohalogenbenzols der allgemeinen Formel II

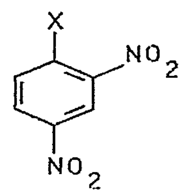

(II)

wobei X = -Cl oder -Br bedeutet, mit einem Alkalimetallalkoholat der allgemeinen Formel III

ROM ... (III)

wobei R die bereits eingangs genannte Bedeutung besitzt und M ein Alkalimetallkation bedeutet, in Gegenwart eines Lösungsmittels ist dadurch gekennzeichnet, daß es bei Temperaturen von -25°C bis +50°C in einem unpolaren, inerten Lösungsmittel durchgeführt wird.

Das erfindungsgemäße Verfahren wird vorzugsweise bei Temperaturen von -5 bis +25°C, und ganz besonders bevorzugt von 0 bis +7°C, durchgeführt.

Von den Verbindungen der Formel II wird das 2,4-Dinitrochlorbenzol bevorzugt.

Für M kann ein beliebiges Alkalimetallkation, wie z.B. $Li^+$, $Na^+$, $K^+$, $Rb^+$ stehen, von denen im allgemeinen $Na^+$ aus Kostengründen bevorzugt ist. Das zum Einsatz kommende Alkalimetallalkoholat ROM kann in Substanz eingesetzt werden. Es kann aber auch vor der eigentlichen Reaktion in dem zur Anwendung kommenden inerten unpolaren Lösungsmittel, in an sich bekannter Weise, aus dem Alkohol ROH und dem das Alkalimetallkation M liefernden Alkalimetall, wobei R und M die bereits genannte Bedeutung besitzen, hergestellt werden.

Normalerweise wird dabei das Alkalimetall in dem zur Anwendung kommenden unpolaren, inerten Lösungsmittel vorgelegt und geschmolzen und/oder fein verteilt. Hierzu wird dann der Alkohol ROH zudosiert, wobei der Alkohol auch in dem unpolaren inerten Lösungsmittel gelöst sein kann. In der Regel beträgt dabei das Molverhältnis zwischen dem Alkalimetall und dem Alkohol ROH = 1 : 1, doch können auch Alkoholüberschüsse bis zu einem Molverhältnis von 1 : 1,25 zur Anwendung kommen.

Das erfindungsgemäße Verfahren wird in einem Lösungsmittel durchgeführt, das unter den Reaktionsbedingungen gegenüber den Ausgangsprodukten der Formeln II und III und gegenüber dem Endprodukt der Formel I und, falls das Ausgangsprodukt der Formel III aus Alkalimetall und Alkohol ROH hergestellt wird, auch gegenüber dem Alkalimetall inert ist.

Die benutzten Lösungsmittel sind darüberhinaus unpolar, d.h. sie besitzen im Gegensatz zu z.B. Alkoholen, Estern oder Nitrilen kein oder ein nur geringes elektrisches Dipolmoment im Bereich von 0 bis $3{,}3 \times 10^{-30}$ Cm (Cm = Coulomb x Meter; die früher benutzte Einheit D = Debye ist veraltet, $3{,}3 \times 10^{-30}$ Cm sind etwa 0,99 D).

Geeignete unpolare inerte Lösungsmittel in diesem Sinne sind z.B. aromatische Kohlenwasserstoffe, wie z.B. Benzol, Alkylbenzole, wie z.B. Toluol, Xylole, Ethylbenzol oder cycloalkylkondensierte Aromaten, wie z.B. Tetralin. Auch Gemische aus zwei oder mehr der genannten Lösungsmittel können verwendet werden. Die Lösungsmittel kommen zweckmäßigerweise wasserfrei zum Einsatz.

Von dem inerten, nichtpolaren Lösungsmittel wird mindestens soviel verwendet, daß eine mindestens rührbare Suspension des Reaktionsgemisches entsteht. Hierzu werden bezogen auf 1 Gewichsteil des 2,4-Dinitrohalogenobenzols der Formel II etwa 3 bis 5 Gewichtsteile des inerten, nicht polaren Lösungsmittels benötigt. Es ist jedoch zweckmäßig, von dem inerten, nicht polaren Lösungsmittel mehr als diese Mindestmenge zu verwenden, so daß zweckmäßigerweise bezogen auf 1 Gewichtsteil des 2,4-Dinitrohalogenobenzols der Formal II 5 bis 150 Gewichtsteile, vorzugsweise 5 bis 20 Gewichtsteile, des inerten, nicht polaren Lösungsmittels zum Einsatz kommen. Es ist zwar möglich, noch mehr inertes nicht polares Lösungsmittel zu verwenden als dem sogenannten Gewichtsverhältnis 1 : 150 entspricht, doch bringt dies keine Vorteile.

Die Ausgangskomponenten der Formel II und III werden bei den genannten Temperaturen in dem inerten, unpolaren Lösungsmittel oder Lösungsmittelgemisch vereinigt. In der Regel beträgt dabei das Molverhältnis zwischen dem 2,4-Dinitro-chlor-oder -brom-benzol der Formel II und dem Alkalimetallalkoholat der Formel III = 1 : (1 bis 1,2). Höhere Alkalimetallalkoholatüberschüsse, z.B. bis 1 : 1,5, anzuwenden, ist zwar möglich, bringt aber keine Vorteile. Normalerweise werden beide Ausgangskomponenten der Formel II und III zunächst in dem Lösungsmittel oder Lösungsmittelgemisch gelöst oder suspendiert und dann langsam vereinigt. In der Regel wird dabei die Lösung der Verbindung der Formel II vorgelegt, und unter Durchmischung wird die Suspension des Alkalimetallalkoholats der Formel III zudosiert.

Unter Umständen kann die Suspension des Alkalimetallalkoholats durch eine Temperaturerhöhung in eine Lösung überführt und dadurch leichter gleichmäßig dosiert werden. In diesem Fall ist jedoch besonders darauf zu achten, daß die genannten Reaktionstemperaturen nicht überschritten werden.

Die Alkoxy-und Alkyl-gruppen in den für R stehenden Resten können geradkettig oder verzweigt sein. Die Alkalimetallalkoholate der Formel III können sich z.B. von folgenden Alkoholen ableiten:

Methylglykol (= Ethylenglykolmonomethylether, die folgenden Bezeichnungen sind analog gebildet), Ethylglykol, Propylglykol, Isopropylglykol, Butylglykol, Phenylglykol; Methyldiglykol (= Diethylenglykolmonomethylether, folgenden Bezeichnungen sind analog gebildet), Ethyldiglykol, Isopropyldiglykol, Butyldiglykol, Isobutyldiglykol; 2-oder 3-Methoxy-propanol, 2-oder 3-Propoxy-propanol, 2-oder 3-Isopropoxy-propanol, 2-oder 3-Isobutoxy-propanol, 2-, 3-oder 4-Methoxy-butanol, 2-, 3-oder 4-Isopropoxy-butanol, 2-, 3-oder 4-Butoxy-butanol, 2-, 3-oder 4-Isobutoxy-butanol; 4,8-Dioxanonan-1-ol, 4,8-Dioxadecan-1-ol, 4,8-Dioxaundecan-1-ol, 5,10-Dioxaundecan-1-ol, 5,10-Dioxadodecan-1-ol, 3,6-Dioxa-2,5-dimethyl-heptan-1-ol, 3,6-Dioxa-2,5-dimethyl-nonan-1-ol, 3,6-Dioxa-2,5-diethyl-octan-1-ol, Tetrahydrofurfurylalkohol.

Vorzugsweise leitet sich das Alkoholat der Formel III von Methylgkykol - (Ethylenglykolmonomethylether) ab, d.h. es wird für die Verdindung III vorzugsweise ein Alkalimetall-2-methoxy-ethanolat verwendet.

Wenn als Alkalimetallalkoholat der Formel III ein Gemisch mit zwei oder mehr verschiedenen Resten R eingesetzt wird, wobei R die eingangs gennannte Bedeutung besitzt, dann entstehen Gemische aus zwei oder mehr Verbindungen der Formel I.

Das nach Beendigung der Umsetzung anfallende Reaktionsgemisch kann nach einem eventuellen Vernichten eines Alkoholatüberschusses und nach Entfernen der ausgefallenen Alkalimetallsalze entweder ohne Zwischenisolierung für nachfolgende Reaktionen, z.B. für eine katalytische Reduktion, eingesetzt werden oder nach an sich bekannten Verfahren aufgearbeitet werden.

Ein gegebenenfalls vorhandener Alkalimetallalkoholat-überschuß oder -rest . wird durch Zugabe einer äquivalenten Menge einer anorganischen oder organischen Säure, wie z.B. Schwefelsäure, Essigsäure, Oxalsäure oder Benzoesäure, zerstört. Nach Ausrühren mit Wasser und Phasentrennung kann der 2,4-Dinitrophenylether der Formel I durch Abdestillieren des Lösungsmittels, gegebenenfalls unter vermindertem Druck, isoliert werden.

Bei dem erfindungsgemäßen Verfahren werden hohe Ausbeuten an Ethern der Formel I erhalten, die 97 % oder mehr betragen. Die Gehalte an Nebenprodukten, insbesondere an 2,4-Dinitrophenol, liegen unter 2 % und sinken auf vernachlässigbare Werte unter 0,3 % (Dinitrophenol unter 0,1 %), wenn die Reaktion bei Temperaturen unter +25°C durchgeführt wird. Die Ausbeutesteigerung und drastische Reduzierung der Nebenproduktbildung waren, insbesondere in ihren Ausmaßen, nicht vorhersehbar, da bei Umsetzungen von 2,4-Dinitrohalogenbenzolen mit Alkoholaten in unpolaren organischen Lösungsmitteln ein stärkeres Auftreten von Nebenprodukten, wie z.B. von Azoxy-und Azoverbindungen, zu befürchten war.

Gegenüber einer Umsetzung der Alkalimetallalkoholate mit dem 2,4-Dinitrohalogenbenzol in Gegenwart eines polaren organischen Lösungsmittels, wie z.B. in einem geradkettigen oder verzweigten Alkohol, z.B. Methanol oder Ethanol, oder in einem geradkettigen oder verzweigten Alkylacetat oder in einem Glykol oder Glykolether, bietet die Verwendung von unpolaren organischen Lösungsmitteln nach dem erfindungsgemäßen Verfahren z.B. den Vorteil, daß bei der Herstellung des Alkoholats aus dem Alkalimetall und dem Alkohol im großtechnischen Maßstab die Chargierung der ersteren wesentlich einfacher zu gestalten und die Reaktion erheblich leichter unter Kontrolle zu halten ist. Außerdem können Zersetzungen des Alkalimetallalkoholats vollständig vermieden werden. Darüberhinaus bietet die Verwendung eines unpolaren, mit Wasser nicht mischbaren Lösungsmittels Vorteile bei der Aufarbeitung des Reaktionsansatzes bzw. bei nachfolgenden Reaktionen ohne Zwischenisolierung des Produktes.

In den nachfolgenden Beispielen sind Temperaturen in Celsiusgraden angegeben. Sofern nichts anderes angegeben ist, bedeuten Teile Gewichsteile und Prozente Gewichtsprozente.

Beispiel 1

β-Methoxyethoxy-2,4-dinitrobenzol

24,2 g Natrium werden in 1000 ml Xylol aufgeschmolzen. Bei 100°C tropft man 83,6g Methylglykol (= Ethylenglykolmonomethylether) zu und rührt bis zur vollständigen Auflösung des Natriums. Die abgekühlte Suspension wird innerhalb von 2 h bei 0 bis 5°C zu einer Lösung von 202,6 g 2,4-Dinitrochlorbenzol in 400 ml Xylol gegeben. Man rührt weitere 60 min nach.

Dann werden zur Zerstörung des Basenüberschusses 2,5 g $H_2SO_4$ zugegeben und die ausgefallenen Salze (Kochsalz und Natriumsulfat) abgetrennt.

Die so erhaltene Lösung des β-Methoxyethoxy-2,4-dinitrobenzols kann direkt für weitere Umsetzungen eingesetzt werden.

Zur Isolierung der Verbindung werden der Reaktionslösung 2,5 g $H_2SO_4$ und 500 ml warmes Wasser (50°C) zugefügt. Nach Ausrühren und Phasentrennung wird die organische Phase im Wasserstrahlvakuum vom Lösungsmittel befreit.

Man erhält 237, 9 g $\beta$-Methoxyethoxy-2,4-dinitrobenzol (98,3 % der Theorie, bezogen auf 2,4-Dinitrochlorbenzol) als gelben kristallinen Feststoff vom Fp: 36° C. Der gaschromatographisch ermittelte Reingehalt beträgt über 99 %.

Beispiel 2 (Vergleichsbeispiel)

Analog der Reaktionsstufe 1 der EP-PS 0011048 werden 213 g geschmolzenes 2,4-Dinitrobenzol in 632 g Methylglykol eingebracht und unter Rühren gelöst. Zu der gerührten Lösung werden 86 g 50%ige wäßrige NaOH-Lösung bei 65°C zugetropft. Anschließend wird eine Stunde bei 65°C nachgerührt.

Das erhaltene Reaktionsgemisch enthält nach der HPLC-Analyse $\beta$-Methoxyethoxy-2,4-dinitrobenzol und 2,4-Dinitrophenol im Gewichtsverhältnis 73 : 21 sowie 6 Teile nicht näher identifizierte Nebenprodukte. Der Ansatz wird mit 800 g Eis versetzt, und das ausgefallene Produkt wird abgesaugt und mit 2 1 kaltem Wasser gewaschen. Man erhält 193 g (75 % der Theorie) eines dunkelbraunen, Nebenprodukte enthaltenden Öls (gaschromatographisch bestimmter Reingehalt 94 %). Aus der wäßrigen Phase werden durch Ansäuren auf pH 1 36,7 g (19 %) 2,4-Dinitrophenol erhalten.

Beispiel 3 (Vergleichsbeispiel):

Bei einer Wiederholung des Beispiels 2 werden anstelle wäßriger NaOH-Lösung 42 g festes NaOH eingesetzt.

Es werden 229,2 g (=90,2 % der Theorie) $\beta$-Methoxyethoxy-2,4-dinitrobenzol als dunkelbrauner Feststoff vom Fp: 31 bis 33°C erhalten, der einen gaschromatographisch bestimmten Reingehalt von 97 % besitzt. Aus der wäßrigen Phase werden durch Ansäuern auf pH 1 13,5 g (7 %) 2,4-Dinitrophenol isoliert.

Beispiel 4

Bei einer Wiederholung des Beispiels 1 werden statt Methylglykol 152 g Phenylglykol eingesetzt.

Es werden 298,1 g (= 98 % der Theorie) $\beta$-Phenoxyethoxy-2,4-dinitrobenzol vom Fp: 61 bis 62°C mit einem gaschromatograpisch ermittelten Reingehalt von 99,8 % erhalten.

Beispiel 5

Bei einer Wiederholung des Beispiels 1 werden statt des Methylglykols 132,2 g Methyldiglykol (= Diethylenglykolmonomethylether) eingesetzt.

Es werden 278,1 g (= 97,2 % der Theorie) 2-(2-Methoxyethoxy)-ethoxy-2,4-dinitrobenzol vom Fp: 43 bis 45°C und einem gaschromatographisch ermittelten Reingehalt von 99,8 % erhalten.

Beispiel 6

Bei einer Wiederholung des Beispiels 1 werden statt des Methylglykols 114,6 g 3-Methoxy-butanol eingesetzt.

Es werden 262 g (= 97 % der Theorie) (3-Methoxy-butoxy)-2,4-dinitrobenzol vom Fp: 42 bis 43°C und einem gaschromatographisch ermittelten Reingehalt von 99,7 % erhalten.

Beispiel 7

Bei einer Wiederholung des Beispiels 1 werden statt des Methylglykols 112 g Tetrahydrofurfurylalkohol eingesetzt.

Es werden 260 g (= 97 % der Theorie) (Tetrahydro-furfuryloxy)-2,4-dinitrobenzol vom Fp:52 bis 54°C und einer gaschromatographisch ermittelten Reinheit von 99,8 % erhalten.

Beispiel 8 bis 12

In analoger Weise wie in Beispiel 1 werden Methylglykol (= Ethylenglykolmonomethylether) und 2,4-Dinitrochlorbenzol umgesetzt. In der nachfolgenden Tabelle sind das benutzte Alkalimetall M, das Molverhältnis zwischen Alkalimetall : Methylglykol : 2,4-Dinitrochlorbenzol, das Lösungsmittel, die Reaktionstemperatur und die Reaktionszeit angegeben. Dabei werden die in der Tabelle angegebenen Ausbeuten an $\beta$-Methoxyethoxy-2,4-dinitrobenzol erhalten. In Schmelzpunkt und Reingehalt entsprechen die Produkte dem nach Beispiel 1 erhaltenen Material.

T A B E L L E

| Nr. | M | Molverhältnis | Lösungsmittel | Reaktionstemp. in °C | Reaktions-Zeit in h | Ausbeute in % |
|---|---|---|---|---|---|---|
| 8 | Li | 1,1 : 1,15 : 1 | Xylol | 0 - 5 | 3 | 98,6 |
| 9 | K | 1,1 : 1,15 : 1 | Xylol | 0 - 5 | 3 | 98,0 |
| 10 | Na | 1,2 : 1,3 : 1 | Toluol | 40 | 2 | 97,8 |
| 11 | Na | 1,2 : 1,5 : 1 | Toluol | -20 | 5 | 98,1 |
| 12 | Na | 1,0 : 1,1 : 1 | Ethylbenzol | 25 | 3 | 98,2 |

**Ansprüche**

1. Verfahren zur Herstellung von 2,4-Dinitrophenylethern der allgemeinen Formel I

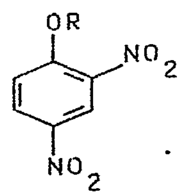

(I)

wobei

R = ($C_1$-$C_4$)Alkoxy-($C_2$-$C_4$)alkyl, Phenoxy-($C_2$-$C_4$)-alkyl, ($C_1$-$C_4$)Alkoxy-($C_2$-$C_4$)alkoxy-($C_2$-$C_4$)alkyl oder Tetrahydrofuryl

bedeutet, durch Umsetzung eines 2,4-Dinitrohalogenbenzols der allgemeinen Formel II

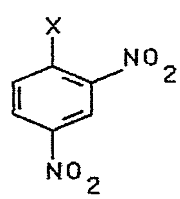

(II)

wobei X = -Cl oder -Br bedeutet, mit einem Alkalimetallalkoholat der allgemeinen Formel III

ROM ... (III)

wobei R die bereits genannte Bedeutung besitzt und M ein Alkalimetallkation bedeutet, in Gegenwart eines Lösungsmittels, dadurch gekennzeichnet, daß die Umsetzung bei einer Temperatur von -25°C bis +50°C in einem unpolaren, inerten Lösungsmittel durchgeführt wird.

2. Verfahren nach Anspruch 1, dadurch gekennzeichnet, daß das Molverhältnis von 2,4-Dinitrohalogenbenzol der Formel II und dem Alkalimetallalkoholat der Formel III 1 : (1 bis 1,2) beträgt.

3. Verfahren nach Anspruch 1 und/oder 2, dadurch gekennzeichnet, daß es bei Temperaturen von -5 bis +25°C durchgeführt wird.

4. Verfahren nach einem oder mehreren der Ansprüche 1 bis 3, dadurch gekennzeichnet, daß es bei Temperaturen von 0 bis +7°C durchgeführt wird.

5. Verfahren nach einem oder mehreren der Ansprüche 1 bis 4, dadurch gekennzeichnet, daß eine Lösung des 2,4-Dinitrohalogenbenzols der Formel II mit einer Lösung oder Suspension des Alkalimetallalkoholats der Formel III vereinigt wird.

6. Verfahren nach einem oder mehreren der Ansprüche 1 bis 5, dadurch gekennzeichnet, daß eine Lösung des 2,4-Dinitrohalogenbenzols der Formel II vorgelegt und eine Lösung oder Suspension des Alkalimetallalkoholats der Formel III zugesetzt wird.

7. Verfahren nach einem oder mehreren der Ansprüche 1 bis 6, dadurch gekennzeichnet, daß als 2,4-Dinitrohalogenbenzol der Formel II 2,4-Dinitrochlorbenzol verwendet wird.

8. Verfahren nach einem oder mehreren der Ansprüche 1 bis 7, dadurch gekennzeichnet, daß als Alkalimetallalkoholat der Formel III ein Alkalimetall-2-methoxy-ethanolat verwendet wird.

9. Verfahren nach einem oder mehreren der Ansprüche 1 bis 8, dadurch gekennzeichnet, daß ein Alkalimetallalkoholat der Formel III verwendet wird, wobei M für Na steht.

10. Verfahren nach einem oder mehreren der Ansprüche 1 bis 9, dadurch gekennzeichnet, daß ein inertes Lösungsmittel verwendet wird, das ein Dipolmoment von 0 bis $3{,}3 \times 10^{-30}$ Cm besitzt.